# EUROPEAN PATENT APPLICATION

(11) **EP 4 837 499 A1**
(43) Date of publication of application: **11.11.2026**
(21) Application number: 26177360.0
(22) Date of filing: 07.05.2026
(51) Int. Cl.: A61B 1/06, A61B 1/00, A61B 1/05

(54) **HEAD END STRUCTURE AND ENDOSCOPE**

(30) Priority: 08.05.2025 CN 202510588455
(71) Applicant: Shenzhen HugeMed Medical Technical Development Co., Ltd., Shenzhen, Guangdong 518112 (CN)
(72) Inventor: CHEN, Chuang, Shenzhen, Guangdong, 518112 (CN); ZHANG, Guoqiang, Shenzhen, Guangdong, 518112 (CN); ZHAI, Huiwen, Shenzhen, Guangdong, 518112 (CN); HU, Jifan, Shenzhen, Guangdong, 518112 (CN); XIAO, Xiaofan, Shenzhen, Guangdong, 518112 (CN); LI, Wenzhen, Shenzhen, Guangdong, 518112 (CN)
(74) Representative: Balder IP Law, S.L.

(57) **Abstract**

Disclosed are a head end structure and an endoscope. The head end structure includes a head end base (1), a first window (2), and a second window (3). A lens end of the head end base (1) is provided with a first mounting cavity (11) and a second mounting cavity (12). The first mounting cavity (11) is configured for a camera assembly to be disposed in. The second mounting cavity (12) is configured for an illumination assembly to be disposed in. The first window (2) is sealingly connected to the head end base (1) and covers the first mounting cavity (11). The second window (3) is sealingly connected to the head end base (1) and covers the second mounting cavity (12).

## Description

### TECHNICAL FIELD

The present invention relates to the technical field of medical instruments and, in particular, to a head end structure and an endoscope.

### BACKGROUND

A medical endoscope is a precision medical instrument used for directly observing internal cavities or organs of the human body. It is a medical instrument that integrates optical imaging, mechanical manipulation, and electronic technology and enters the body through natural orifices or small incisions, enabling a surgeon to observe internal structures in real time and perform operations such as diagnosis, treatment, or minimally invasive surgery.

A head end base of the medical endoscope is a core structural component at the front end of the endoscope, mainly responsible for optical imaging, instrument guidance, function control, and the like. Currently, an illumination assembly and a camera assembly are mounted on the head end base of the medical endoscope. However, in the related art, the illumination assembly and the camera assembly are easily adhered to by mucus during surgery, thereby affecting a surgeon's surgical operation.

### SUMMARY

The present invention provides a head end structure and an endoscope to solve the problem in the related art that an illumination assembly and a camera assembly on a head end base are easily adhered to by mucus during surgery and further affect a surgeon's surgical operation.

In one aspect, the present invention provides a head end structure. The head end structure is configured for a camera assembly and an illumination assembly to be mounted on. The head end structure includes a head end base, a first window, and a second window.

A lens end of the head end base is provided with a first mounting cavity and a second mounting cavity. The first mounting cavity is configured for the camera assembly to be disposed in. The second mounting cavity is configured for the illumination assembly to be disposed in.

The first window is sealingly connected to the head end base and covers the first mounting cavity. The first window is configured for isolating the camera assembly from an outside of the first mounting cavity.

The second window is sealingly connected to the head end base and covers the second mounting cavity. The second window is configured for isolating the illumination assembly from an outside of the second mounting cavity.

In an optional solution of the preceding head end structure, the first window includes a first head end portion and a first side end portion connected to each other. Part of the first mounting cavity penetrates a side end of the head end base. In the first mounting cavity, the first head end portion is sealingly connected to the lens end of the head end base, and the first side end portion is sealingly connected to the side end of the head end base.

In an optional solution of the preceding head end structure, a side of the first head end portion facing the head end base protrudes from the head end base. A junction between the first head end portion and the lens end and a junction between the first head end portion and the first side end portion are each formed as an inclined slope.

In an optional solution of the preceding head end structure, an inner wall of the first mounting cavity recesses to form a first embedding groove and a second embedding groove intersecting each other. The first side end portion has a first embedding portion and a second embedding portion intersecting each other. The first embedding portion is embedded in the first embedding groove. The second embedding portion is embedded in the second embedding groove.

In an optional solution of the preceding head end structure, the second window includes a second head end portion and a second side end portion connected to each other. Part of the second mounting cavity penetrates a side end of the head end base. In the second mounting cavity, the second head end portion is sealingly connected to the lens end of the head end base, and the second side end portion is sealingly connected to the side end of the head end base.

In an optional solution of the preceding head end structure, the lens end of the head end base is further provided with a positioning groove. One end of the positioning groove communicates with the second mounting cavity. The second head end portion is provided with a positioning protrusion protruding from the second head end portion. The positioning protrusion is embedded in the positioning groove.

In an optional solution of the preceding head end structure, an inner wall of the second mounting cavity recesses to form a third embedding groove and a fourth embedding groove intersecting each other. The second side end portion has a third embedding portion and a fourth embedding portion intersecting each other. The third embedding portion is embedded in the third embedding groove. The fourth embedding portion is embedded in the fourth embedding groove.

In an optional solution of the preceding head end structure, the head end base is a PEEK base. The first window and the second window are each a PC window.

In an optional solution of the preceding head end structure, the first window and the second window are both integrally formed with the head end base by secondary injection molding.

In another aspect, the present invention provides an endoscope. The endoscope includes the preceding head end structure.

The head end structure includes the head end base, the first window, and the second window. The lens end of the head end base is provided with the first mounting cavity and the second mounting cavity. The camera assembly is disposed in the first mounting cavity. The illumination assembly is disposed in the second mounting cavity. Therefore, the camera assembly and a related circuit can be accommodated in the first mounting cavity, and the illumination assembly and a related circuit can be accommodated in the second mounting cavity. The first window is sealingly connected to the head end base and covers the first mounting cavity. The first window is used for isolating the camera assembly from the outside. The second window is sealingly connected to the head end base and covers the second mounting cavity. The second window is used for isolating the illumination assembly from the outside. Therefore, with the arrangement of the first window and the second window, mucus generated during surgery can be blocked without affecting the normal operation of the camera assembly and the illumination assembly, thereby preventing mucus from adhering to the camera assembly and the illumination assembly and affecting a surgeon's surgical operation when the head end base is open. Moreover, such an arrangement prevents the camera assembly and the illumination assembly from scratching a patient when the head end base is open.

### BRIEF DESCRIPTION OF DRAWINGS

FIG. 1 is a structure view of a head end structure according to an embodiment of the present invention.
FIG. 2 is a partially disassembled view of the head end structure according to an embodiment of the present invention.
FIG. 3 is a first disassembled section view of the head end structure according to an embodiment of the present invention.
FIG. 4 is a second disassembled section view of the head end structure according to an embodiment of the present invention.

### Reference list

- 1: head end base
- 11: first mounting cavity
- 111: first embedding groove
- 112: second embedding groove
- 12: second mounting cavity
- 121: third embedding groove
- 122: fourth embedding groove
- 13: positioning groove
- 14: working channel
- 2: first window
- 21: first head end portion
- 22: first side end portion
- 221: first embedding portion
- 222: second embedding portion
- 3: second window
- 31: second head end portion
- 311: positioning protrusion
- 32: second side end portion
- 321: third embedding portion
- 322: fourth embedding portion

### DETAILED DESCRIPTION

The technical solutions of the present invention are described clearly and completely below in conjunction with the drawings. Apparently, the embodiments described below are part, not all, of embodiments of the present invention. Based on embodiments of the present invention, all other embodiments obtained by those of ordinary skill in the art without creative work are within the scope of the present invention.

In the description of the present invention, it should be understood that the orientational or positional relationships indicated by terms "center", "above", "below", "left", "right", "vertical", "horizontal", "inside", "outside" and the like are based on the orientational or positional relationships illustrated in the drawings, which are for the mere purpose of facilitating and simplifying the description of the present invention, and these relationships do not indicate or imply that the device or component referred to has a specific orientation and is constructed and operated in a specific orientation, and thus it is not to be construed as limiting the present invention. In addition, terms such as "first" and "second" are used only for the purpose of description and are not to be construed as indicating or implying relative importance. The terms "first position" and "second position" are two different positions. Moreover, when a first feature is described as "on", "above", or "over" a second feature, the first feature is right on, above, or over the second feature, the first feature is obliquely on, above, or over the second feature, or the first feature is simply at a higher level than the second feature. When the first feature is described as being "under", "below", or "underneath" the second feature, the first feature is right under, below, or underneath the second feature, the first feature is obliquely under, below, or underneath the second feature, or the first feature is simply at a lower level than the second feature.

In the description of the present invention, it is to be noted that unless otherwise expressly specified and limited, the term "mounted", "connected to each other", or "connected" should be construed in a broad sense, for example, as securely connected, detachably connected, or integrally connected; mechanically connected or electrically connected; directly connected to each other or indirectly connected to each other via an intermediary; or interconnected between two elements. For those of ordinary skill in the art, specific meanings of the preceding terms in the present invention may be understood based on specific situations.

Embodiments of the present invention are described below in detail. Examples of the embodiments are illustrated in the drawings, where the same or similar reference numerals throughout the drawings represent the same or similar elements or elements having the same or similar functions. The embodiments described below with reference to the drawings are illustrative and intended to explain the present invention and are not to be construed as limiting the present invention.

This embodiment provides a head end structure. A camera assembly and an illumination assembly are able to be mounted on the head end structure.

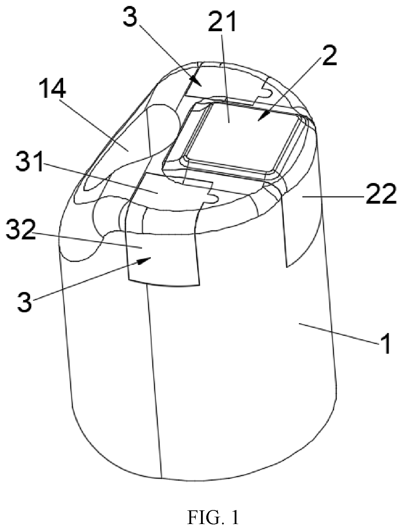

As shown in FIGS. 1 to 4, the head end structure includes a head end base 1, a first window 2, and a second window 3. A lens end of the head end base 1 is provided with a first mounting cavity 11 and a second mounting cavity 12. The camera assembly is disposed in the first mounting cavity 11. The illumination assembly is disposed in the second mounting cavity 12. Therefore, the camera assembly and a related circuit can be accommodated in the first mounting cavity 11, and the illumination assembly and a related circuit can be accommodated in the second mounting cavity 12. The first window 2 is sealingly connected to the head end base 1 and covers the first mounting cavity 11. The first window 2 is used for isolating the camera assembly from the outside. The second window 3 is sealingly connected to the head end base 1 and covers the second mounting cavity 12. The second window 3 is used for isolating the illumination assembly from the outside. Therefore, with the arrangement of the first window 2 and the second window 3, mucus generated during surgery can be blocked without affecting the normal operation of the camera assembly and the illumination assembly, thereby preventing mucus from adhering to the camera assembly and the illumination assembly and affecting a surgeon's surgical operation when the head end base 1 is open. Moreover, such an arrangement prevents the camera assembly and the illumination assembly from scratching a patient when the head end base 1 is open.

Specifically, the head end base 1 is further provided with a working channel 14. The working channel 14 and the first mounting cavity 11 are located on two opposite sides of the head end base 1. Moreover, two second mounting cavities 12 are provided. The connection line of the two second mounting cavities 12 is arranged diagonally to the connection line of the first mounting cavity 11 and the working channel 14 so that the space of the head end base 1 can be reasonably used, facilitating a compact design of the head end structure.

Further, the head end base 1 is a PEEK base. Therefore, the head end base 1 is made of a PEEK material to utilize the high strength, high wear resistance and good biocompatibility of the PEEK material to guarantee the structural strength and durability of the head end base 1 without causing an adverse reaction in the human body, resulting in high medical safety. The first window 2 and the second window 3 are each a PC window. Therefore, the first window 2 and the second window 3 are both made of a PC material to utilize the high transparency of the PC material to facilitate light transmission and the observation of the patient's internal conditions. Moreover, the PC material also has a certain strength, toughness, and self-lubricating capacity, effectively reducing mucus adhesion. The first window 2 and the second window 3 are both integrally formed with the head end base 1 by secondary injection molding. Therefore, with the arrangement of the head end base 1 made of the PEEK material and the first window 2 and second window 3 made of the PC material, the connection strength and sealing performance between the first window 2 and the head end base 1 and between the second window 3 and the head end base 1 are guaranteed after secondary injection molding.

Specifically, the first window 2 is an optical window whose structure complies with optical design principles. It not only protects the internal camera assembly but also enhances the visual experience. Moreover, physical isolation exists on the optical path between the first window 2 and the second window 3. Optical protection against stray light also exists between the first window 2 and the second window 3.

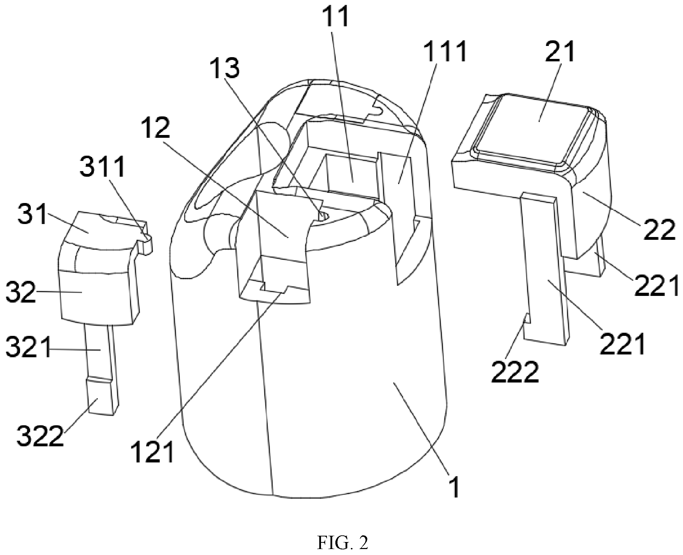

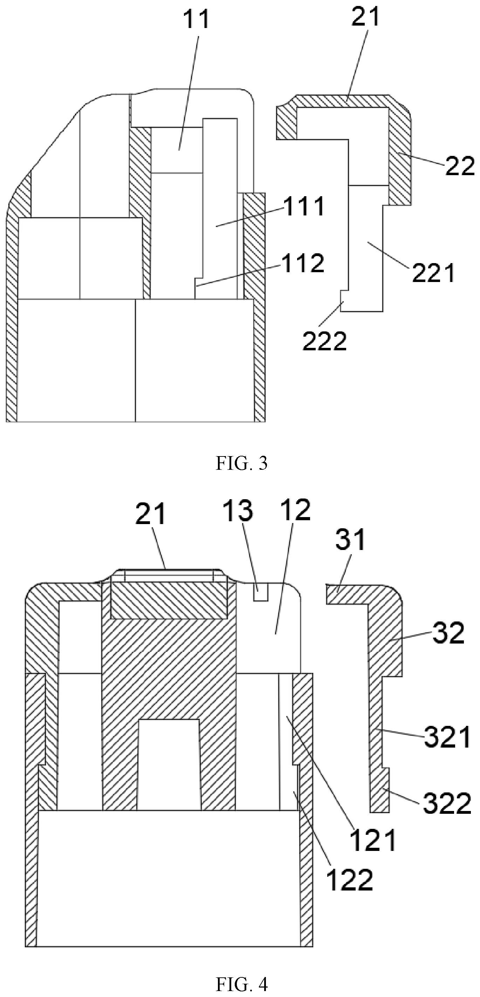

As shown in FIGS. 2 and 3, the first window 2 includes a first head end portion 21 and a first side end portion 22 connected to each other. Part of the first mounting cavity 11 penetrates a side end of the head end base 1. In the first mounting cavity 11, the first head end portion 21 is sealingly connected to the lens end of the head end base 1, and the first side end portion 22 is sealingly connected to the side end of the head end base 1. Therefore, when the camera assembly performs shooting through the lens end of the head end base 1, the shooting can also be performed through the side end of the head end base 1, thereby increasing the shooting range and facilitating the surgeon's operation and use. A side of the first head end portion 21 facing the head end base 1 protrudes from the head end base 1. A junction between the first head end portion 21 and the lens end and a junction between the first head end portion 21 and the first side end portion 22 are each formed as an inclined slope. Therefore, the outer periphery of the first head end portion 21 can form a flow guide surface, allowing mucus to be detached from the first head end portion 21 under the guidance of the flow guide surface and thereby preventing mucus from accumulating on the first window 2 and affecting the normal operation of the camera assembly.

Optionally, an inner wall of the first mounting cavity 11 recesses to form a first embedding groove 111 and a second embedding groove 112 intersecting each other. The first side end portion 22 has a first embedding portion 221 and a second embedding portion 222 intersecting each other. The first embedding portion 221 is embedded in the first embedding groove 111. The second embedding portion 222 is embedded in the second embedding groove 112. Therefore, through the cooperation of the first embedding portion 221 with the first embedding groove 111 and the cooperation of the second embedding portion 222 with the second embedding groove 112, the stability of the first window 2 mounted on the head end base 1 can be guaranteed, preventing the first window 2 from vibrating and affecting the operation of the camera assembly.

As shown in FIGS. 2 and 4, the second window 3 includes a second head end portion 31 and a second side end portion 32 connected to each other. Part of the second mounting cavity 12 penetrates the side end of the head end base 1. In the second mounting cavity 12, the second head end portion 31 is sealingly connected to the lens end of the head end base 1, and the second side end portion 32 is sealingly connected to the side end of the head end base 1. Therefore, when the illumination assembly performs illumination through the lens end of the head end base 1, the illumination can also be performed through the side end of the head end base 1, thereby increasing the illumination range and facilitating the surgeon's operation and use. The lens end of the head end base 1 is further provided with a positioning groove 13. One end of the positioning groove 13 communicates with the second mounting cavity 12. The second head end portion 31 is provided with a positioning protrusion 311 protruding from the second head end portion 31. The positioning protrusion 311 is embedded in the positioning groove 13. Through the cooperation of the positioning protrusion 311 with the positioning groove 13, the mounting of the second window 3 on the head end base 1 can be positioned.

Optionally, an inner wall of the second mounting cavity 12 recesses to form a third embedding groove 121 and a fourth embedding groove 122 intersecting each other. The second side end portion 32 has a third embedding portion 321 and a fourth embedding portion 322 intersecting each other. The third embedding portion 321 is embedded in the third embedding groove 121. The fourth embedding portion 322 is embedded in the fourth embedding groove 122. Therefore, through the cooperation of the third embedding portion 321 with the third embedding groove 121 and the cooperation of the fourth embedding portion 322 with the fourth embedding groove 122, the stability of the second window 3 mounted on the head end base 1 can be guaranteed, preventing the second window 3 from vibrating and affecting the operation of the illumination assembly.

This embodiment further provides an endoscope. The endoscope includes the preceding head end structure. With the preceding head end structure used in the endoscope, mucus generated during surgery can be blocked without affecting the normal operation of the camera assembly and the illumination assembly, thereby preventing mucus from adhering to the camera assembly and the illumination assembly and affecting a surgeon's surgical operation when the head end base 1 is open. Moreover, such an arrangement prevents the camera assembly and the illumination assembly from scratching a patient when the head end base 1 is open.

## Claims

1. A head end structure, configured for a camera assembly and an illumination assembly to be mounted on, and the head end structure comprises:
a head end base (1), wherein a lens end of the head end base (1) is provided with a first mounting cavity (11) and a second mounting cavity (12), the first mounting cavity (11) is configured for the camera assembly to be disposed in, and the second mounting cavity (12) is configured for the illumination assembly to be disposed in;
a first window (2), wherein the first window (2) is sealingly connected to the head end base (1) and covers the first mounting cavity (11), and the first window (2) is configured for isolating the camera assembly from an outside of the first mounting cavity (11); and
a second window (3), wherein the second window (3) is sealingly connected to the head end base (1) and covers the second mounting cavity (12), and the second window (3) is configured for isolating the illumination assembly from an outside of the second mounting cavity (12).

2. The head end structure according to claim 1, wherein the first window (2) comprises a first head end portion (21) and a first side end portion (22) connected to each other; part of the first mounting cavity (11) penetrates a side end of the head end base (1); and in the first mounting cavity (11), the first head end portion (21) is sealingly connected to the lens end of the head end base (1), and the first side end portion (22) is sealingly connected to the side end of the head end base (1).

3. The head end structure according to claim 2, wherein a side of the first head end portion (21) facing the head end base (1) protrudes from the head end base (1), and a junction between the first head end portion (21) and the lens end and a junction between the first head end portion (21) and the first side end portion (22) are each formed as an inclined slope.

4. The head end structure according to claim 2, wherein an inner wall of the first mounting cavity (11) recesses to form a first embedding groove (111) and a second embedding groove (112) intersecting each other, the first side end portion (22) has a first embedding portion (221) and a second embedding portion (222) intersecting each other, the first embedding portion (221) is embedded in the first embedding groove (111), and the second embedding portion (222) is embedded in the second embedding groove (112).

5. The head end structure according to claim 1, wherein the second window (3) comprises a second head end portion (31) and a second side end portion (32) connected to each other; part of the second mounting cavity (12) penetrates a side end of the head end base (1); and in the second mounting cavity (12), the second head end portion (31) is sealingly connected to the lens end of the head end base (1), and the second side end portion (32) is sealingly connected to the side end of the head end base (1).

6. The head end structure according to claim 5, wherein the lens end of the head end base (1) is further provided with a positioning groove (13), one end of the positioning groove (13) communicates with the second mounting cavity (12), the second head end portion (31) is provided with a positioning protrusion (311) protruding from the second head end portion (31), and the positioning protrusion (311) is embedded in the positioning groove (13).

7. The head end structure according to claim 5, wherein an inner wall of the second mounting cavity (12) recesses to form a third embedding groove (121) and a fourth embedding groove (122) intersecting each other, the second side end portion (32) has a third embedding portion (321) and a fourth embedding portion (322) intersecting each other, the third embedding portion (321) is embedded in the third embedding groove (121), and the fourth embedding portion (322) is embedded in the fourth embedding groove (122).

8. The head end structure according to any one of claims 1 to 7, wherein the head end base (1) is a PEEK base, and the first window (2) and the second window (3) are each a PC window.

9. The head end structure according to claim 8, wherein the first window (2) and the second window (3) are both integrally formed with the head end base (1) by secondary injection molding.

10. An endoscope, comprising the head end structure according to any one of claims 1 to 9.
